# FASCICULE DE BREVET EUROPEEN

(11) **EP 0 927 152 B1**
(45) Date de publication et mention de la délivrance du brevet: **12.12.2001**
(21) Numéro de dépôt: 97940207.0
(22) Date de dépôt: 10.09.1997
(51) Int. Cl.: C07C 213/00, C07C 215/30, C07B 53/00, C07B 41/02

(54) **PROCEDE DE PREPARATION DE BENZYLALCOOLS ALPHA-SUBSTITUES OPTIQUEMENT ACTIFS**
VERFAHREN ZUR HERSTELLUNG VON OPTISCH AKTIVEN ALPHA-SUBSTITUIERTEN BENZYLALKOHOLEN
METHOD FOR PREPARING OPTICALLY ACTIVE ALPHA-SUBSTITUTED BENZYLALCOHOLS

(30) Priorité: 11.09.1996 FR 9611065
(43) Date de publication de la demande: 07.07.1999
(73) Titulaire: SANOFI-SYNTHELABO, 75013 Paris (FR)
(72) Inventeur: AGBOSSOU, Francine, F-62820 Libercourt (FR); DEVOCELLE, Marc, F-59000 Lille (FR); DORMOY, Jean-Robert, F-04200 Sisteron (FR); MORTREUX, André, F-59510 Hem (FR)
(74) Mandataire: Gillard, Marie-Louise
(86) Numéro de dépôt international: FR9701592
(87) Numéro de publication internationale: WO9811053

(56) Documents cités:
- EP-A- 0 253 700
- EP-A- 0 443 923
- TAMIO HAYASHI ET AL.: "asymmetric synthesis of 2-amino-1-arylethanols by catalytic asymmetric hydrogenation" TETRAHEDRON LETTERS, no. 5, 1979, OXFORD GB, pages 425-428, XP002028704
- MIHALY NOGRADI: "Stereoselective Synthesis" 1987 , VCH , WEINHEIM DE, XP002028705 voir page 53 - page 89

## Description

La présente invention concerne un procédé pour la préparation de benzylalcools α-substitués optiquement actifs à partir des cétones correspondantes.

Les benzylalcools α-substitués optiquement actifs sont des intermédiaires clé dans la synthèse de nombreux produits pharmaceutiques, notamment dans la préparation des phényléthanolamines actives sur les récepteurs β-adrénergiques.

Une des approches de synthèse pour ces produits est la réduction asymétrique des cétones à l'aide de catalyseurs chiraux. De tels catalyseurs chiraux sont pas exemple décrits dans Organometallics, 15: 2440-2449, 1996; dans Synlett, Apr. 1995, 358-360; dans EP-A-0 253 700 et dans EP-A-0 443 923.

Ces documents décrivent des classes de complexes de métaux du groupe VIII, notamment du rhodium, contenant, entre autres, des ligands chiraux; plus particulièrement, ils décrivent les complexes où les composants sont liés à l'atome du métal par des liaisons "covalentes".

L'article T. Hayashi et al., Tetrahedron Letters, 1979, 5, 425-428 décrit la synthèse asymétrique de dérivés 2-amino-1-aryl-éthanols par hydrogénation asymétrique catalytique en présence d'un complexe hydroalkylferrocenylphosphine de rhodium. Les catalyseurs décrits dans cet article sont des catalyseurs non-hétérocycliques et contenant du fer.

La publication M. Nogradi, « Stereoselective synthesis », 1987, VCH, Weinheim DE, 53-89 mentionne des complexes phosphiniques de rhodium utilisés comme catalyseurs d'hydrogénation.

Il a été maintenant trouvé que des complexes du type "ionique" de rhodium sont des catalyseurs très performants dans la préparation de benzylalcools α-substitués optiquement actifs.

Ainsi, la présente invention concerne un procédé de préparation d'un alcool optiquement actif de formule (I) 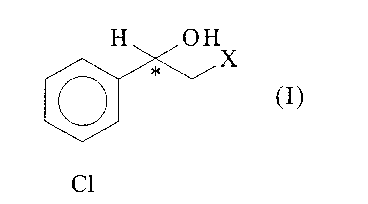 dans laquelle l'atome de carbone indiqué par le symbole * peut avoir la configuration *(R)* ou (*S*) et X représente un groupe amino non substitué ou un groupe mono- ou di- (C₁-C₄)alkylamino éventuellement salifiés, caractérisé en ce que
a/ on soumet une cétone de formule (II) 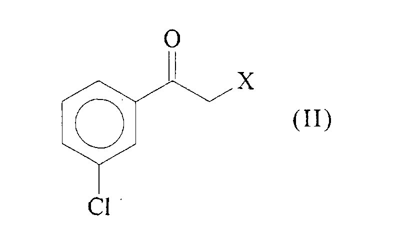 dans laquelle X est tel que défini ci-dessus, à une hydrogénation catalytique en présence d'un complexe de rhodium de formule (III)

   [Rh(AMPP)(1,5-COD)]⁺ A⁻ (III)

   dans laquelle (AMPP) représente un aminophosphine-phosphinite chiral de formule (IV) 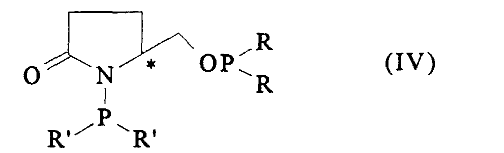 où l'atome de carbone indiqué par le symbole * peut avoir la configuration (R) ou (*S*), R et R' représentent un cyclohexyle ou un cyclopentyle, (1,5-COD) représente le 1,5-octadiène et A⁻ représente un anion monovalent peu coordinant et stériquement encombré;
b/ on isole l'alcool optiquement actif de formule (I).

Le terme "(C₁-C₄)alkyle" désigne un résidu alkylique linéaire ou ramifié contenant 1 à 4 atomes de carbone tel que le méthyle, l'éthyle, le n-propyle, le i-propyle, le n-butyle, le i-butyle, le s-butyle et le t-butyle.

Le terme "anion monovalent monovalent peu coordinant et stériquement encombré" selon la présente invention désigne un anion tel que le perchlorate, le tétrafluoroborate, le tétraphénylborate ou l'hexafluorophosphate. Un anion particulièrement préféré est le tétrafluoroborate.

Les groupes amino non substitué ou mono- ou di-(C₁-C₄)-alkylamino peuvent être salifiés par exemple par une molécule d'acide chlorhydrique.

Selon un aspect préféré, la présente invention concerne un procédé pour la préparation d'un alcool optiquement actif de formule (I') 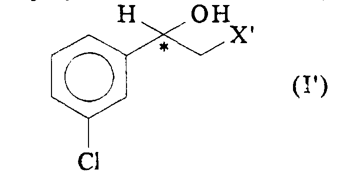 dans laquelle X' représente un groupe amino non substitué ou diméthylamino salifiés par une molécule d'acide chlorhydrique, caractérisé en ce que
a/ on soumet une cétone de formule (II') 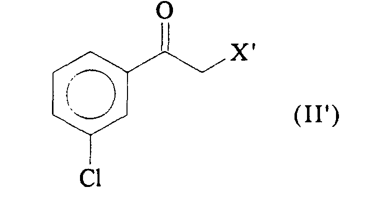 dans laquelle X' est tel que défini ci-dessus, à une hydrogénation catalytique en présence d'un complexe de rhodium de formule (III')

   [Rh(AMPP)(1,5-COD)]⁺BF₄ ⁻ (III')

   dans laquelle (AMPP) est tel que défini ci-dessus;
b/ on isole l'alcool optiquement actif de formule (I').

La configuration absolue des alcools de formule (I) est déterminée par la configuration de l'aminophosphine-phosphinite qu'on utilise. Lorsque l'on utilise un aminophosphine-phosphinite à configuration (*S*) on obtient sélectivement les alcools à configuration (*S*), et lorsque l'on emploie un aminophosphine-phosphinite à configuration (*R*) on obtient les alcools à configuration (*R*).

La réaction d'hydrogénation asymétrique selon la présente invention est conduite dans un solvant protique tel que par exemple un alcool, comme le méthanol ou l'éthanol.

La température de réaction est généralement comprise entre -30°C et le reflux du solvant de réaction, de préférence entre 20°C et 50°C.

L'hydrogénation de la présente invention peut être effectuée à la pression atmosphérique ou sous pression; avantageusement la réaction est conduite à une pression d'hydrogène comprise entre 1 et 80 bar, notamment entre 20 et 50 bar.

La quantité de catalyseur à utiliser est comprise entre 0,005 et 2 % en moles par rapport à la cétone de départ, de préférence entre 0,1 et 1 %, avantageusement environ 0,5%.

Les temps de réaction, même s'ils varient en fonction des autres paramètres de réaction, sont très brefs; en général un délai de quelques heures est suffisant pour la conversion de 100% du produit de départ.

Les alcools de formules (I) et (I') sont isolés selon les techniques usuelles, par exemple par évaporation du solvant après filtration du catalyseur.

En effet, les catalyseurs "ioniques" de formule (III), notamment ceux de formule (III') sont plus performants des catalyseurs "covalents" utilisés selon EP-A-0 443 923 et dans Synlett, Apr. 1995, 358-360, dans la réduction asymétrique de la présente invention. Plus particulièrement, l'utilisation des catalyseurs de formule (III) et notamment (III') selon la présente invention permet de réduire énormement les temps de réaction, élément qui, surtout en travaillant au niveau industriel, constitue un important avantage.

Les cétones de départ de formule (II) sont des composés connus.

Les catalyseur de formule (III) et notamment (III') sont préparés comme décrit dans EP-A-0 443 923 et dans Synlett, Apr. 1995, 358-360.

Le procédé de la présente invention permet l'obtention des formes énantiomériques des alcools de formule (I) pratiquement pures, en général présentant un excès énantiomérique supérieur à 95%.

Les alcools des formule (I) très purs ainsi obtenus peuvent donc être utilisés en tant que produits de départ pour la préparation des phényléthanolamines à action β-adrénergique, par exemple les alcools de formule (I) à configuration (*R*) sont utiles dans la synthèse de l'ester éthylique de l'acide {(7S)-7-[(2R)-2-(3-chlorophényl)-2-hydroxyéthylamino]-5,6,7,8-tétrahydronaphtalèn-2-yloxy}-acétique, composé à l'étude pour son activité agoniste sur les récepteurs β₃-adrénergiques.

Les exemples qui suivent illustrent l'invention.

### MODE OPERATOIRE

On dissout le chlorhydrate de la cétone de formule (II) dans du méthanol. On introduit la solution dans un autoclave et on la met sous azote. On dissout le catalyseur de formule (III) dans du méthanol et on le transfert, sous azote, dans l'autoclave à l'aide d'une canule. On met sous vide l'autoclave puis sous pression d'hydrogène (50 bar) suivie d'une dépressurisation jusqu'à 3-5 bar. On répète l'opération deux fois avant la mise sous pression d'hydrogène définitive. On met en route l'agitation et on laisse réagir. Après dépressurisation de l'autoclave, on évapore le solvant du brut réactionnel. On extrait le produit de réaction en traitant le solide par 10 ml d'eau. On filtre le catalyseur sur fritté, on ajoute de l'éthanol au filtrat pour faciliter l'évaporation de l'eau et on évapore le solvant sous pression réduite. On obtient le composé de formule (I).
Le tableau I montre les résultats des exemples 1 à 3.
La détermination de l'excès énantiomérique de l'alcool de formule (I) où X est un groupe amino non substitué, est conduite sur le dérivé N-benzoylé, préparé sur un échantillon de produit selon la méthode suivante:
On agite à la température ambiante pendant 2,5 heures 78,6 mg (0,4 mmol) de composé amino avec 53,1 mg (0,4 mmol) de chlorure de benzoyle et 84 mg (2,2 mmol) de triéthylamine dans 10 ml de tétrahydrofurane. On élimine le chlorhydrate de triéthylamine par filtration et on évapore le résidu à l'évaporateur rotatif. On obtient un solide blanc.

| Ex. | cétone de formule (II) | AMPP de formule (IV) isomère (*S*) | Conditions de réaction | | | conversion | rendement | alcool de formule (I) isomère (*S*) e.e. |
|---|---|---|---|---|---|---|---|---|
| | | | bar H₂ | temp. °C | temps h. | | | |
| 1 | X = -NMe₂·HCl | R=R'=cyclopentyle | 50 | 20 | 2 | 100 % | 83 % | 96 %* |
| 2 | X = -NMe₂·HCl | R=R'=cyclohexyle | 50 | 20 | 2 | 98 % | 86 % | 96 %* |
| 3 | X = -NH₃Cl | R=R'=cyclopentyle | 50 | 20 | 1,5 | 100 % | 80 % | 95 % ** |

| | | | | | | | | |
|---|---|---|---|---|---|---|---|---|
| Les excès énantiomérique sont évalués par HPLC sur colonne Chiralcel (Daicel): * élution: alcool isopropylique/hexane: 5/95; 0,4 ml/min (base libre). | | | | | | | | |
| ** élution: alcool isopropylique/hexane: 10/90; 0,6 ml/min (produit N-benzoylé). | | | | | | | | |

## Revendications

1. Procédé de préparation d'un alcool optiquement actif, de formule (I) 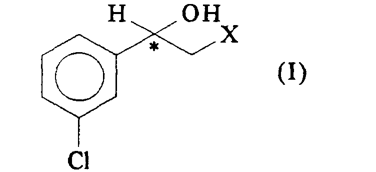 dans laquelle l'atome de carbone indiqué par le symbole * peut avoir la configuration (*R*) ou (*S*) et X représente un groupe amino non substitué ou un groupe mono- ou di-(C₁-C₄)alkylamino, éventuellement salifiés, **caractérisé en ce que**
a/ on soumet une cétone de formule (II) 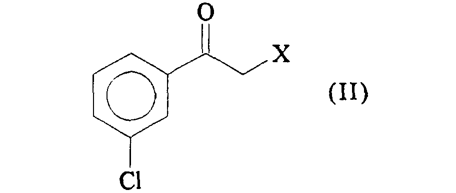 dans laquelle X est tel que défini ci-dessus, à une hydrogénation catalytique en présence d'un complexe de rhodium de formule (III)
[Rh(AMPP)(1,5-COD)]⁺A⁻ (III)
dans laquelle (AMPP) représente un aminophosphine-phosphinite chirale de formule (IV) 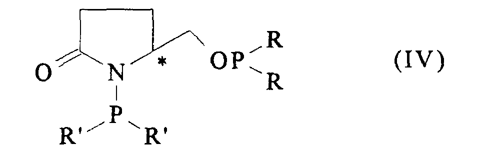 où l'atome de carbone indiqué par le symbole * peut avoir la configuration (*R*) ou (*S*), R et R' représentent indépendemment un cyclohexyle ou un cyclopentyle et (1,5-COD) représente le 1,5-octadiène et A⁻ représente un anion monovalent peu coordinant et stériquement encombré;
b/ on isole l'alcool optiquement actif de formule (I).

2. Procédé selon la revendication 1 pour la préparation d'un alcool optiquement actif de formule (I') 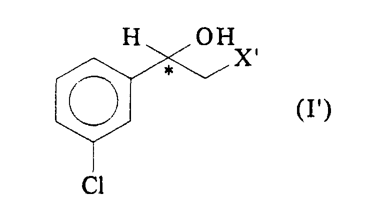 dans laquelle X' représente un groupe amino non substitué ou diméthylamino salifiés par une molécule d'acide chlorhydrique, **caractérisé en ce que**
a/ on soumet une cétone de formule (II') 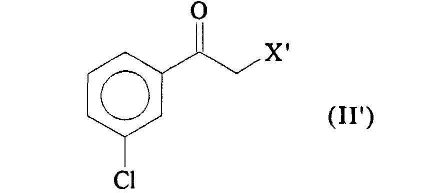 dans laquelle X' est tel que défini ci-dessus, à une hydrogénation catalytique en présence d'un complexe de rhodium de formule (III')
[Rh(AMPP)(1,5-COD)]⁺BF₄ ⁻ (III')
dans laquelle (AMPP) est tel que défini ci-dessus;
b/ on isole l'alcool optiquement actif de formule (I').

3. Procédé selon les revendications 1 ou 2 **caractérisé en ce qu'**on utilise un aminophosphine-phosphinite à configuration (*S*) pour obtenir l'alcool de formule (I) à configuration (*S*).

4. Procédé selon les revendications 1 ou 2 **caractérisé en ce qu'**on utilise un aminophosphine-phosphinite à configuration (*R*) pour obtenir l'alcool de formule (I) à configuration (*R*).

5. Procédé selon la revendication 1 **caractérisé en ce que** la température de réaction est comprise entre -30°C et la température de reflux du solvant.

6. Procédé selon la revendication 1 **caractérisé en ce que** la température de réaction est comprise entre 20°C et 50°C.

7. Procédé selon la revendication 1 **caractérisé en ce que** la réaction est conduite sous une pression d'hydrogène comprise entre 1 et 80 bar.

8. Procédé selon la revendication 1 **caractérisé en ce que** la quantité de catalyseur est comprise entre 0,005 et 2 % en moles par rapport à la cétone de départ, de préférence entre 0,1 et 1 %, avantageusement environ 0,5%.

9. Procédé selon la revendication 1 caractérisé en ce X est un groupe diméthylamino salifié par une molécule d'acide chlorhydrique.

10. Procédé selon la revendication 1 caractérisé en ce X est un groupe amino non substitué salifié par une molécule d'acide chlorhydrique.

## Patentansprüche

1. Verfahren zur Herstellung eines optisch aktiven Alkohols der Formel (I) 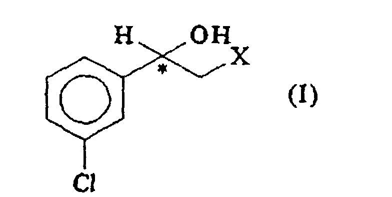 worin das mit dem Symbol * versehene Kohlenstoffatom (R)- oder (S)-Konfiguration haben kann und X eine unsubstituierte Aminogruppe oder eine Mono- oder Di-(C₁-C₄)-alkylaminogruppe, gegebenenfalls in Salzform, darstellt, **dadurch gekennzeichnet, dass**
a/ ein Keton der Formel (II) 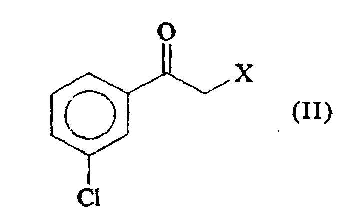 worin X wie oben definiert ist, einer katalytischen Hydrierung in Anwesenheit eines Rhodiumkomplexes der Formel (III)
[Rh(AMPP)(1,5-COD)]⁺A⁻ (III)
unterzogen wird, worin (AMPP) ein chirales Aminophosphinphosphinit der Formel (IV) 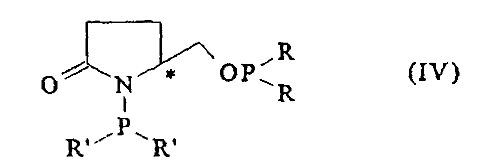 darstellt, worin das mit dem Symbol * versehene Kohlenstoffatom (R)- oder (S)- Konfiguration haben kann, R und R' unabhängig voneinander ein Cyclohexyl oder ein Cyclopentyl darstellen und (1,5-COD) 1,5-Octadien darstellt und A⁻ ein kaum koordinativ anlagerndes und sterisch voluminöses monovalentes Anion darstellt;
b/ der optisch aktive Alkohol der Formel (I) isoliert wird.

2. Verfahren nach Anspruch 1 zur Herstellung eines optisch aktiven Alkohols der Formel (I') 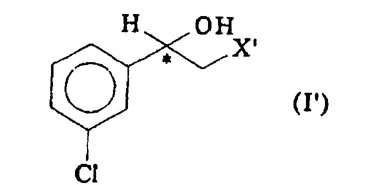 worin X' eine unsubstituierte Aminogruppe oder Dimethylamino, durch ein Salzsäuremolekül in Salzform übergeführt, darstellt, **dadurch gekennzeichnet, dass**
a/ ein Keton der Formel (II') 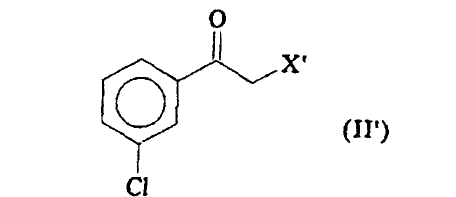 worin X' wie oben definiert ist, einer katalytischen Hydrierung in Anwesenheit eines Rhodiumkomplexes der Formel (III')
[Rh(AMPP)(1,5-COD)]⁺BF₄ ⁻ (III')
worin (AMPP) wie oben definiert ist, unterzogen wird;
b/ der optisch aktive Alkohol der Formel (I') isoliert wird.

3. Verfahren nach Anspruch 1 oder 2, **dadurch gekennzeichnet, dass** ein Aminophosphinphosphinit mit (S)-Konfiguration zur Gewinnung des Alkohols der Formel (I) mit (S)- Konfiguation verwendet wird.

4. Verfahren nach Anspruch 1 oder 2, **dadurch gekennzeichnet, dass** ein Aminophosphinphosphinit mit (R)-Konfiguration zur Gewinnung des Alkohols der Formel (I) mit (R)- Konfiguation verwendet wird.

5. Verfahren nach Anspruch 1, **dadurch gekennzeichnet, dass** die Reaktionstemperatur zwischen -30 °C und der Rückflusstemperatur des Lösungsmittels liegt.

6. Verfahren nach Anspruch 1, **dadurch gekennzeichnet, dass** die Reaktionstemperatur zwischen 20 °C und 50 °C beträgt.

7. Verfahren nach Anspruch 1, **dadurch gekennzeichnet, dass** die Reaktion unter einem Wasserstoffdruck zwischen 1 und 80 bar durchgeführt wird.

8. Verfahren nach Anspruch 1, **dadurch gekennzeichnet, dass** die Menge an Katalysator zwischen 0,005 und 2 Mol-%, bezogen auf das Ausgangsketon, vorzugsweise zwischen 0,1 und 1 %, vorteilhaft etwa 0,5 %, beträgt.

9. Verfahren nach Anspruch 1, **dadurch gekennzeichnet, dass** X eine durch ein Salzsäuremolekül in Salzform übergeführte Dimethylaminogruppe ist.

10. Verfahren nach Anspruch 1, **dadurch gekennzeichnet, dass** X eine unsubstituierte, durch ein Salzsäuremolekül in Salzform übergeführte Aminogruppe ist.

## Claims

1. A method of preparing an optically active alcohol of formula (I): 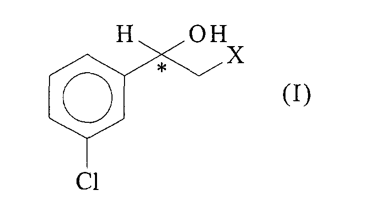 in which the carbon atom indicated by the symbol * can have the (*R*) or (*S*) configuration and X represents a non-substituted amino group or a mono- or di- (C₁-C₄)alkylamino group, which are optionally salified, **characterised in that**:
a/ a ketone of formula (II): 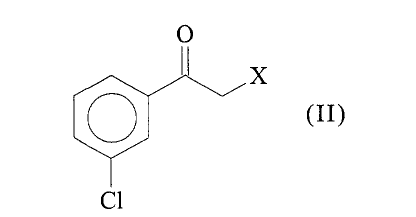 in which X is as defined above, is subjected to a catalytic hydrogenation in the presence of a rhodium complex of formula (III) :
[Rh(AMPP)(1.5-COD)]⁺A⁻ (III)
in which (AMPP) represents a chiral aminophosphine-phosphinite of formula (IV) : 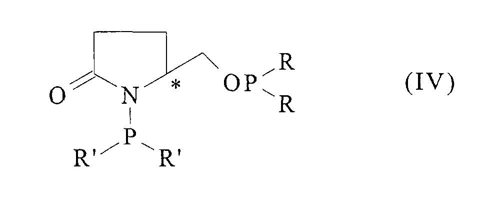 where the carbon atom indicated by the symbol * can have the (*R*) or (*S*) configuration, R and R' independently represent a cyclohexyl or a cyclopentyl and (1.5-COD) represents 1.5-octadiene and A represents a sterically hindered and little-co-ordinating monovalent anion ; and
b/ the optically active alcohol of formula (I) is isolated.

2. The method according to claim 1 for preparing an optically active alcohol of formula (I'): 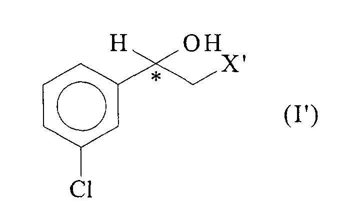 in which X' represents a non-substituted amino group or a dimethylamino group, which are salified with a molecule of hydrochloric acid, **characterised in that**:
a/ a ketone of formula (II'): 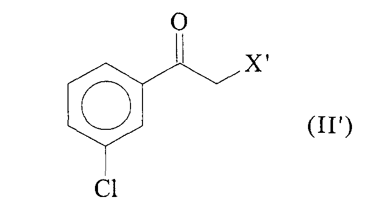 in which X' is as defined above, is subjected to a catalytic hydrogenation in the presence of a rhodium complex of formula (III'):
[Rh(AMPP)(1.5-COD)]⁺BF₄ ⁻ (III')
in which (AMPP) is as defined above ; and
b/ the optically active alcohol of formula (I') is isolated.

3. The method according to claim 1 or 2, **characterised in that** an aminophosphine-phosphinite of (*S*) configuration is used to give the alcohol of formula (I) of (*S*) configuration.

4. The method according to claim 1 or 2, **characterised in that** an aminophosphine-phosphinite of *(R)* configuration is used to give the alcohol of formula (I) of *(R)* configuration.

5. The method according to claim 1, **characterised in that** the reaction temperature is between -30°C and the reflux temperature of the solvent.

6. The method according to claim 1, **characterised in that** the reaction temperature is between 20°C and 50°C.

7. The method according to claim 1, **characterised in that** the reaction is conducted under a pressure of hydrogen between 1 and 80 bar.

8. The method according to claim 1, **characterised in that** the amount of catalyst is between 0.005 and 2 mole % with respect to the starting ketone, preferably between 0.1 and 1 %, advantageously about 0.5%.

9. The method according to claim 1, **characterised in that** X is a dimethylamino group salified with a molecule of hydrochloric acid.

10. The method according to claim 1, **characterised in that** X is a non-substituted amino group salified with a molecule of hydrochloric acid.
